# EUROPEAN PATENT APPLICATION

(11) **EP 4 296 269 A1**
(43) Date of publication of application: **27.12.2023**
(21) Application number: 22756195.8
(22) Date of filing: 16.02.2022
(51) Int. Cl.: C07D 409/12, A61K 9/10, A61K 31/496, A61K 47/06, A61P 25/04, A61P 25/06, A61P 25/18, A61P 25/20, A61P 25/22, A61P 25/24, A61P 25/28

(54) **AMORPHOUS FORM AND COMPOSITION CONTAINING SAID AMORPHOUS FORM**

(30) Priority: 16.02.2021 WO PCT/JP2021/005721
(71) Applicant: Otsuka Pharmaceutical Co., Ltd., Tokyo 101-8535 (JP)
(72) Inventor: KAMADA, Naoki, Osaka-shi, Osaka 540-0021 (JP); YOSHIMURA, Motoyasu, Osaka-shi, Osaka 540-0021 (JP); KIMOTO, Mariko, Osaka-shi, Osaka 540-0021 (JP)
(74) Representative: Hoffmann Eitle
(86) International application number: PCT/JP2022/006064
(87) International publication number: WO 2022/176877

(57) **Abstract**

Provided are an amorphous solid dispersion containing compound (I), an amorphous form containing compound (I) and an organic acid, an amorphous solid dispersion containing the amorphous form, a pharmaceutical composition containing the amorphous form or the amorphous solid dispersion, and preparation methods for these.

## Description

### Technical Field

The present disclosure relates to an amorphous form containing 7-[4-(4-benzo[b]thiophen-4-yl-piperazin-1-yl)butoxy]-1H-quinolin-2-one, and a composition containing the amorphous form. The disclosures of all documents cited in the present specification are incorporated herein by reference.

### Background Art

7-[4-(4-benzo[b]thiophen-4-yl-piperazin-1-yl)butoxy]-1H-quinolin-2-one ("compound (I)" or "brexpiprazole" below) has dopamine D₂ receptor partial agonist activity, serotonin 5-HT_{2A} receptor antagonist activity, and adrenergic α₁ receptor antagonist activity. In addition to those activities, compound (I) is also known to have serotonin uptake inhibition activity (or serotonin reuptake inhibition activity ) together, and thus has a broad therapeutic spectrum for central neurological diseases, in particular, schizophrenia (PTL 1). Brexpiprazole is also known as a drug that is less soluble in water (poorly soluble drug). Because poorly soluble drugs are also less soluble in the gastrointestinal tract, their poor absorption through the gastrointestinal mucosa can be a problem. Thus, even now it is still an important technical issue to design formulations so as to improve the solubility and oral absorption of poorly soluble drugs in order to develop the medicinal benefits of such poorly soluble drugs.

### Citation List

### Patent Literature

PTL 1: JP2006-316052A

### Summary of Invention

### Technical Problem

An object of the present invention is to provide an amorphous solid dispersion containing compound (I) excellent in dissolution, further preferably excellent in stability, an amorphous form containing compound (I) and an organic acid, an amorphous solid dispersion containing the amorphous form, a pharmaceutical composition containing the amorphous form or the amorphous solid dispersion, and methods for preparing these.

### Solution to Problem

The present inventors found that an amorphous solid dispersion of compound (I) that exhibits excellent dissolution can be obtained by adding a specific polymer to compound (I).

Additionally, the inventors also found that an amorphous form containing compound (I) and an organic acid can be obtained, and that an amorphous solid dispersion containing compound (I) and an organic acid exhibits excellent dissolution, preferably excellent stability, of compound (I).

The present disclosure includes, for example, the subject matter described in the following items.
Item 1.
   An amorphous form comprising 7-[4-(4-benzo[b]thiophen-4-yl-piperazin-1-yl)butoxyJ-1H-quinolin-2-one and at least one organic acid.
Item 2.
   The amorphous form according to Item 1, wherein the organic acid is a carboxylic acid.
Item 2a.
   The amorphous form according to Item 1, wherein the organic acid is a monocarboxylic acid, a dicarboxylic acid, or a tricarboxylic acid.
Item 2b.
   The amorphous form according to Item 1, 2, or 2a, wherein the organic acid is a carboxylic acid having 1 to 8 carbon atoms.
Item 2c.
   The amorphous form according to Item 1, 2, 2a, or 2b, wherein the organic acid is at least one carboxylic acid selected from the group consisting of acetic acid, lactic acid, malic acid, citric acid, oxalic acid, tartaric acid, propionic acid, malonic acid, succinic acid, glutaric acid, adipic acid, fumaric acid, maleic acid, and phthalic acid.
Item 2d.
   The amorphous form according to Item 1, 2, 2a, 2b, or 2c, wherein the organic acid is at least one carboxylic acid selected from the group consisting of acetic acid, lactic acid, malic acid, tartaric acid, propionic acid, malonic acid, succinic acid, glutaric acid, adipic acid, fumaric acid, and phthalic acid.
Item 3.
   The amorphous form according to Item 1, 2, 2a, 2b, 2c, or 2d, wherein the organic acid is lactic acid.
Item 3a.
   The amorphous form according to Item 1, 2, 2a, 2b, 2c, 2d, or 3, wherein the ratio by mass of the 7-[4-(4-benzo[b]thiophen-4-yl-piperazin-1-yl)butoxy]-1H-quinolin-2-one to the organic acid is within the range of 100:0.1 to 60.
Item 4.
   An amorphous solid dispersion comprising the amorphous form of Item 1, 2, 2a, 2b, 2c, 2d, 3, or 3a and at least one enteric polymer.
Item 5.
   An amorphous solid dispersion comprising 7-[4-(4-benzo[b]thiophen-4-yl-piperazin-1-yl)butoxy]-1H-quinolin-2-one and at least one enteric polymer.
Item 6.
   The amorphous solid dispersion according to Item 4 or 5, wherein the enteric polymer is a nonionic water-soluble polymer.
Item 7.
   The amorphous solid dispersion according to any one of Items 4 to 6, wherein the enteric polymer is at least one selected from the group consisting of hydroxypropyl methylcellulose, derivatives thereof, and polyvinylpyrrolidone. Item 7a.
   The amorphous solid dispersion according to any one of Items 4 to 7, wherein the enteric polymer is hypromellose acetate succinate. Item 7b.
   The amorphous solid dispersion according to Item 4, 5, 6, 7, or 7a, wherein the ratio by mass of the 7-[4-(4-benzo[b]thiophen-4-yl-piperazin-1-yl)butoxy]-1H-quinolin-2-one to the enteric polymer is within the range of 100:10 to 500.
Item 8.
   The amorphous form according to Item 1, 2, 2a, 2b, 2c, 2d, 3, or 3a, or the amorphous solid dispersion according to Item 4, 5, 6, 7, 7a, or 7b, which is a spray-dried powder.
Item 8a.
   The amorphous solid dispersion according to Item 4, 5, 6, 7, 7a, 7b, or 8, wherein in an HPLC chromatogram obtained after the 7-[4-(4-benzo[b]thiophen-4-yl-piperazin-1-yl)butoxy]-1H-quinolin-2-one is stored at 40°C under closed conditions for 4 weeks, the proportion of the area of the peak area of 8-(1-benzothiophen-4-yl)-8-aza-5-azoniaspiro[4.5]decane is 0.1% or lower of the total area of the peak areas of the 7-[4-(4-benzo[b]thiophen-4-yl-piperazin-1-yl)butoxy]-1H-quinolin-2-one and a degradation product thereof taken as 100%.
Item 9.
   A method for producing the amorphous form of any one of Items 1 to 3, or the amorphous solid dispersion of Item 4, 5, 6, 7, 7a, 7b, 8, or 8a, the method comprising
   subjecting to spray drying a mixture containing 7-[4-(4-benzo[b]thiophen-4-yl-piperazin-1-yl)butoxyJ-1H-quinolin-2-one, and
   at least one selected from the group consisting of an enteric polymer and an organic acid.
Item 10.
   A pharmaceutical composition comprising
   at least one selected from the group consisting of the amorphous form of Item 1, 2, or 3 and the amorphous solid dispersion of Item 4, 5, 6, 7, 7a, 7b, 8, or 9, and
   a hydrophilic polymer.
Item 10a.
   The pharmaceutical composition according to Item 10, comprising the 7-[4-(4-benzo[b]thiophen-4-yl-piperazin-1-yl)butoxyJ-1H-quinolin-2-one in an amount within the range of 5 mg to 60 mg.
Item 10b.
   The pharmaceutical composition according to Item 10 or 10a, comprising the amorphous form and/or the amorphous solid dispersion in an amount within the range of 10 to 40% by mass based on a weight of a core tablet (uncoated tablet).
Item 11.
   The pharmaceutical composition according to Item 10, 10a, or 10b, wherein the hydrophilic polymer is at least one selected from the group consisting of cellulose-based water-soluble polymers, polyalkylene oxides, polyalkylene glycols, and polyvinyl alcohols.
Item 11a.
   The pharmaceutical composition according to Item 10, 10a, 10b, or 11, wherein the hydrophilic polymer is at least one selected from the group consisting of hydroxypropyl methylcellulose, hydroxypropyl cellulose, and methylcellulose.
Item 11b.
   The pharmaceutical composition according to Item 10, 10a, 10b, 11, or 11a, comprising the hydrophilic polymer in an amount within the range of 30 to 90% by mass based on a weight of a core tablet (uncoated tablet).
Item 12.
   The pharmaceutical composition according to Item 10, 10a, 10b, 11, 11a, or 11b, which is an oral solid pharmaceutical composition.
Item 13.
   The pharmaceutical composition according to Item 12, wherein after oral administration of the pharmaceutical composition to a human, the steady-state blood concentration of the 7-[4-(4-benzo[b]thiophen-4-yl-piperazin-1-yl)butoxy]-1H-quinolin-2-one is maintained within the range of 15 ng/mL to 400 ng/mL for 1 week.
Item 14.
   The pharmaceutical composition according to Item 10, 10a, 10b, 11, 11a, 11b, 12, or 13, for use in the administration of the 7-[4-(4-benzo[b]thiophen-4-yl-piperazin-1-yl)butoxy]-1H-quinolin-2-one at a dose of 5 mg to 60 mg once a week.
Item 15.
   The pharmaceutical composition according to Item 10, 10a, 10b, 11, 11a, 11b, 12, 13, or 14, for use in the prevention or treatment of a central neurological disease.
Item 15a.
   The pharmaceutical composition according to Item 10, 10a, 10b, 11, 11a, 11b, 12, 13, 14, or 15, for use in the prevention or treatment of at least one central neurological disease selected from the group consisting of schizophrenia, treatment-resistant, refractory, or chronic schizophrenia, schizoaffective disorder, psychotic disorder, mood disorder, bipolar disorder, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder.

### Advantageous Effects of Invention

The present invention provides an amorphous form of compound (I) and an amorphous solid dispersion of compound (I) that are suitable for use in a pharmaceutical composition, in particular, in a sustained-release oral pharmaceutical composition, with excellent dissolution, further preferably excellent stability.

Examples of the disease that is responsive to compound (I) or a salt thereof include schizophrenia, such as treatment-resistant, refractory, or chronic schizophrenia, schizoaffective disorder, psychotic disorder, mood disorder, bipolar disorder (e.g., bipolar I disorder and bipolar II disorder), depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, and acute stress disorder), somatoform disorder (e.g., hysteria, somatization disorder, conversion disorder, pain disorder, and hypochondria), factitious disorder, dissociative disorder, sexual disorder (e.g., sexual dysfunction, libido disorder, sexual arousal disorder, and erectile dysfunction), eating disorder (e.g., anorexia nervosa and bulimia nervosa), sleep disorder, adjustment disorder, substance-related disorder (e.g., alcohol abuse, alcohol intoxication and drug addiction, amphetamine addiction, and narcotism), anhedonia (e.g., loss of pleasure, anhedonia, iatrogenic anhedonia, anhedonia of a psychic or mental cause, anhedonia associated with depression, anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease, and other neurodegenerative diseases, cognitive impairment in schizophrenia, cognitive impairment caused by treatment-resistant, refractory, or chronic schizophrenia, vomiting, motion sickness, obesity, migraine, pain, mental retardation, autistic disorder (autism), Tourette's syndrome, tic disorder, attention deficit hyperactivity disorder, conduct disorder, Down syndrome, impulsive symptoms associated with dementia (e.g., agitation associated with Alzheimer's disease), borderline personality disorder, etc.; and various other CNS diseases.

### Brief Description of Drawings

Fig. 1 shows the results of the study on the dissolution rate of amorphous solid dispersions containing compound (I) prepared by using various enteric polymers.
Fig. 2 shows the results of measuring the dissolution rate of compound (I) from amorphous solid dispersions obtained from compound (I), an enteric polymer, and various organic acids.
Fig. 3 shows the results of analyzing an amorphous solid dispersion of compound (I) and an amorphous solid dispersion containing compound (I) and lactic acid by X-ray diffractometry.
Fig. 4a shows the results of TGA analysis of each amorphous solid dispersion obtained from compound (I), an enteric polymer, and lactic acid.
Fig. 4b shows the results of DSC analysis of amorphous solid dispersions each obtained from compound (I), an enteric polymer, and lactic acid.
Fig. 5 shows the results of NMR measurement of amorphous solid dispersions and their starting materials.
Fig. 6 shows the results of measuring the dissolution rate of compound (I) from hydrogel matrix tablets (with enteric coating) containing amorphous solid dispersions each obtained from compound (I), an enteric polymer, and lactic acid.

### Description of Embodiments

The following describes in more detail embodiments encompassed by the present disclosure. The present disclosure includes, but is not limited to, an amorphous solid dispersion containing compound (I), an amorphous form containing compound (I) and an organic acid, an amorphous solid dispersion containing the amorphous form, a pharmaceutical composition containing the amorphous form or the amorphous solid dispersion, and methods for preparing these. The present disclosure includes all matter disclosed in the present specification and recognizable to those skilled in the art.

As described above, the amorphous form encompassed by the present disclosure contains compound (I) and an organic acid. In the present specification, the amorphous form may be referred to as "the amorphous form of the present disclosure."

The organic acid is preferably a carboxylic acid, and more preferably a monocarboxylic acid, a dicarboxylic acid, or a tricarboxylic acid. Hydroxy acid (also referred to as "hydroxycarboxylic acid") is also preferable. For example, a carboxylic acid with 1 to 8 (1, 2, 3, 4, 5, 6, 7, or 8) carbon atoms is preferable. The carboxylic acid may also be substituted with at least one (e.g., 1, 2, or 3) -OH group. The carboxylic acid may also have one aromatic ring (in particular, a benzene ring) or heterocyclic ring in its structure. The carboxylic acid may be linear or branched, and saturated or unsaturated.

More specifically, the organic acid may be, for example, acetic acid, lactic acid, malic acid, citric acid, oxalic acid, tartaric acid, propionic acid, malonic acid, succinic acid, glutaric acid, adipic acid, fumaric acid, maleic acid, or phthalic acid, with lactic acid being particularly preferable.

The organic acid may be used singly, or in a combination of two or more. The combination of two or more organic acids for use is particularly preferably a combination of citric acid, tartaric acid, and fumaric acid.

The amorphous solid dispersion encompassed by the present disclosure contains the amorphous form of the present disclosure and an enteric polymer. In the present specification, the amorphous solid dispersion may be referred to as "the amorphous solid dispersion of the present disclosure."

The enteric polymer can be any polymer that does not dissolve at low pH and dissolves at near-neutral pH (e.g., a pH of 5 to 6) or at a pH higher than such a pH. For example, polymers that dissolve at a pH of 5, 5.5, 6.0, 6.5, or higher (preferably polymers that do not dissolve at a pH of lower than these pH values) are preferred. Specific examples include nonionic water-soluble polymers, preferably hypromellose (hydroxypropyl methylcellulose), derivatives thereof, and polyvinylpyrrolidone.

In the present specification, derivatives of hypromellose are preferably an ester of hypromellose and a carboxylic acid (preferably a monocarboxylic acid or a dicarboxylic acid). Examples of carboxylic acids include acetic acid, lactic acid, malic acid, citric acid, oxalic acid, tartaric acid, propionic acid, malonic acid, succinic acid, glutaric acid, adipic acid, fumaric acid, maleic acid, and phthalic acid. Of these carboxylic acids, a single carboxylic acid may form an ester, or a combination of two or more carboxylic acids, may form an ester. Examples of preferred esters of hypromellose and a carboxylic acid include hypromellose acetate succinate, and hypromellose phthalate.

The amorphous solid dispersion of the present disclosure can be prepared, for example, by subjecting a mixture containing compound (I), an organic acid, and an enteric polymer to spray drying (spray-drying treatment). The thus-obtained amorphous solid dispersion contains an amorphous form containing compound (I) and an organic acid (i.e., the amorphous form of the present disclosure).

A preferred example of mixtures subjected to spray-drying treatment is a composition prepared by dissolving compound (I), an organic acid, and an enteric polymer in an organic solvent. The organic solvent is preferably, for example, a mixture of dichloromethane and ethanol. The ratio by mass of dichloromethane to ethanol of the mixture is preferably within the range of about 80 to 50:20 to 50, and more preferably about 80 to 60:20 to 40. When a mixture of dichloromethane and ethanol is used as an organic solvent, it is preferable to prepare the mixture for spray-drying treatment, for example, by dissolving an organic acid in ethanol, then adding and mixing dichloromethane, further dissolving an enteric polymer in the solution, and finally adding and dissolving compound (I). The spray-drying treatment can be performed according to a known method.

The ratio by mass of compound (I) to the organic acid contained in the amorphous form or amorphous solid dispersion of the present disclosure is, for example, within the range of about 100:0.1 to 60. The upper limit or the lower limit of the range (0.1 to 60) may be, for example, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, or 59. For example, the range may be about 0.5 to 55 or 1 to 50.

To increase the stability of the amorphous form or amorphous solid dispersion, it is desirable to add as much organic acid as possible to the extent that the salt of the organic acid does not precipitate. For example, when lactic acid is used as an organic acid, the ratio by mass of compound (I) to the organic acid is especially preferably within the range of about 100:30 to 60, and, for example, more preferably about 100:40 to 60.

The ratio by mass of compound (I) to an enteric polymer contained in the amorphous solid dispersion of the present disclosure is, for example, within the range of about 100:10 to 500. The upper limit or the lower limit of the range (10 to 500) may be, for example, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490. For example, the range may be about 20 to 400, or 50 to 300.

The amorphous solid dispersion of the present disclosure is preferably excellent in stability. In the present specification, the phrase "excellent in stability" means, for example, preferably that after the amorphous solid dispersion of the present disclosure is stored at 40°C under closed conditions for 4 weeks, the peak area of 8-(1-benzothiophen-4-yl)-8-aza-5-azoniaspiro[4.5]decane (also referred to as "compound (D)" in the present specification), which is one of the degradation products of compound (I), in a chromatogram obtained by HPLC measurement accounts for 0.1% or lower of the total of the peak areas of compound (I) and the degradation products of compound (I). The above storage conditions are based on the revised version (*Iyakushinpatsu* No. 0603001, June 3, 2003) of the Guidelines for Stability Testing of New Drug Substances and Products (*Iyakushinpatsu* No. 565, issued on May 1, 2001, by the Director of the Evaluation and Licensing Division of the Pharmaceutical and Food Safety Bureau), which were established based on the standards of the International Council for Harmonisation of Technical Requirements for Pharmaceuticals for Human Use (ICH). Humidity is based on section "2.2.7.2. Drug products packaged in impermeable container" in the Guidelines and is not examined in the present application.

In the present specification, the term "degradation products" refers to impurities formed by chemical changes in an active pharmaceutical ingredient during manufacture or in storage of a drug product due to the action of light, heat, pH, and water, or due to reaction with pharmaceutical additives or directly with a container or closure system.

The pharmaceutical composition encompassed by the present disclosure contains the amorphous form of the present disclosure or the amorphous solid dispersion of the present disclosure. In addition to this, the pharmaceutical composition further preferably contains a hydrophilic polymer. In the present specification, the pharmaceutical composition may also be referred to as "the pharmaceutical composition of the present disclosure."

The pharmaceutical composition of the present disclosure is preferably an oral pharmaceutical composition, and is also preferably a solid pharmaceutical composition. More preferably, the pharmaceutical composition of the present disclosure is an oral solid pharmaceutical composition.

The dosage form of the pharmaceutical composition of the present disclosure is not limited. Examples include tablets, pills, powders, granules, gels, capsules, and powdery inhalants.

The hydrophilic polymer for use can be, for example, a cellulose-based water-soluble polymer, a polyalkylene oxide (e.g., polyethylene oxide), a polyalkylene glycol (e.g., polyethylene glycol), a polyvinyl alcohol, etc.

The cellulose-based water-soluble polymer for use can be preferably a cellulose-based water-soluble polymer known in the field of pharmaceutical science. For example, the cellulose-based water-soluble polymer preferably has a structure in which the hydrogen atom of some of the OH groups of cellulose is replaced with a methyl group and/or a hydroxypropyl group. For example, hypromellose (hydroxypropyl methylcellulose) or derivatives of these are preferred. For example, hydroxypropyl cellulose and methylcellulose are preferred.

The hydrophilic polymer may be used singly, or in a combination of two or more.

The hydrophilic polymer for use can also be, for example, a cellulose-based water-soluble polymer with a viscosity of 2.5 to 35,000 mm²/s in the form of a 2% aqueous solution. In particular, a cellulose-based water-soluble polymer with a viscosity of 2.5 to 17.5 mm²/s in the form of a 2% aqueous solution is preferred.

The hydrophilic polymer for use may be the same as or different from the enteric polymer described above. The pharmaceutical composition containing the amorphous solid dispersion of the present disclosure contains the enteric polymer described above. Thus, if the enteric polymer is a hydrophilic polymer, the pharmaceutical composition is encompassed by the pharmaceutical composition containing a hydrophilic polymer.

In particular, when the pharmaceutical composition of the present disclosure is a solid pharmaceutical composition, it is preferred that the pharmaceutical composition contain a mixture of the amorphous form of the present disclosure or the amorphous solid dispersion of the present disclosure with a hydrophilic polymer.

If the pharmaceutical composition of the present disclosure is an oral solid pharmaceutical composition, a particularly preferable embodiment is a sustained-release hydrogel composition. The composition contains the amorphous form or amorphous solid dispersion of the present disclosure as an active component and further contains the hydrophilic polymer described above. A preferable dosage form of the sustained-release hydrogel composition is a hydrogel matrix tablet. The hydrogel matrix tablet is a known technique for controlling drug release with a hydrogel formed from water absorbed from the alimentary tract (after a coating film is dissolved due to a rise in pH after gastric excretion, if the tablet is enteric-coated).

The sustained-release base (hydrogel formation base) for use in the hydrogel matrix tablet can be the hydrophilic polymers described above. More specifically, the sustained-release base can be, for example, a cellulose-based water-soluble polymer, a polyalkylene oxide (e.g., polyethylene oxide), a polyalkylene glycol (e.g., polyethylene glycol), or a polyvinyl alcohol. In the present disclosure, as described above, a cellulose-based polymer is preferably used. If a cellulose-based polymer (e.g., hypromellose) is used as a sustained-release base, the cellulose-based polymer for use preferably has a viscosity of 80 to 35,000 mm²/s in the form of a 2% aqueous solution according to the target dissolution rate.

The sustained-release base may be contained in an amount of, for example, about 30 to 90% by mass or about 50 to 80% by mass based on the mass of the core tablet.

The hydrogel matrix tablet may further contain other additives. Examples of such additives include disintegrators, lubricants, and fluidizers. A preferable example of disintegrators is sodium starch glycolate. The disintegrator may be contained in an amount of, for example, about 10 to 50% by mass or about 20 to 40% by mass based on the mass of the core tablet. A preferable example of lubricants is magnesium stearate. The lubricant may be contained in an amount of, for example, about 0.1 to 5% by mass or about 0.2 to 3% by mass based on the mass of the core tablet. A preferable example of fluidizers is silicon dioxide (in particular, light anhydrous silicic acid). The fluidizer may be contained in an amount of, for example, about 0.1 to 5% by mass or about 0.1 to 3% by mass based on the mass of the core tablet.

The hydrogel matrix tablet is more preferably enteric-coated. For enteric coating, a known enteric coating composition may be used. For example, an enteric coating composition containing an enteric base, such as Eudragit, a plasticizer, such as triethyl citrate, and a lubricant, such as talc, may be preferably used. The enteric coating may be contained in an amount of, for example, about 1 to 40 parts by mass or about 10 to 30 parts by mass, per 100 parts by mass of the core tablet.

Without any limitation, the steady-state blood concentration of compound (I) is preferably maintained, for example, within the range of 15 ng/mL to 400 ng/mL or 50 ng/mL to 300 ng/mL for 1 week after the oral pharmaceutical composition of the present disclosure is perorally administered to a human (in particular, an adult).

Conventional tablets (non-release controlled formulation; 0.5-mg tablets, 1-mg tablets, and 2-mg tablets) containing compound (I) have already been marketed as a therapeutic drug for schizophrenia in many countries including Japan, the United States, and European countries. Conventional tablets have been shown to be safe and effective against central neurological diseases, such as schizophrenia, in clinical trials, and have undergone detailed pharmacokinetic analysis. Given such reports on conventional tablets, it can be understood that any oral pharmaceutical composition that can maintain a steady-state blood concentration of compound (I) within the range of about 15 ng/mL to 400 ng/mL or about 50 ng/mL to 300 ng/mL when administered to humans would be usable in the prevention or treatment of central neurological diseases, such as schizophrenia, in the same manner as the conventional tablets already on the market.

Thus, the oral pharmaceutical composition of the present disclosure may be administered orally, preferably at a frequency lower than once a day (e.g., once a week). The oral pharmaceutical composition of the present disclosure may preferably be administered, one tablet per dose, or two or more tablets per dose (e.g., two, three, four, or five tablets per dose). A person skilled in the art would also be able to appropriately determine the dosage of the oral pharmaceutical composition of the present disclosure to achieve the blood concentration described above referring to the pharmacokinetic information on the conventional tablets described above and the assessment made based on the single-dose protocol and continuous dosing protocol for the oral pharmaceutical composition of the present disclosure. For example, the dosage of compound (I) per dose can be about 5 mg to 60 mg, about 10 mg to 60 mg, about 20 mg to 60 mg, or about 45 mg to 60 mg.

In the present specification, "comprising" also includes the concepts of consisting essentially of and consisting of. The present disclosure also encompasses any combination of the elements described in the specification.

The variety of characteristics (e.g., properties, structures, and functions) described above for embodiments of the present disclosure may be combined in any way in identifying the subject matter encompassed by the disclosure. In other words, the present disclosure encompasses all subject matter formed of any possible combination of the characteristics described in the present specification.

### Examples

The following describes embodiments of the present disclosure in more detail with reference to Examples. However, the embodiments of the disclosure are not limited to the following Examples. Unless otherwise specified below, compound (I) (brexpiprazole) for use was synthesized according to a known method and then milled with a hammer mill.

### Preparation of Amorphous Solid Dispersion of Compound (I) (Brexpiprazole)

6 g of compound (I) and 12 g of an enteric polymer were dissolved in 205.5 g of a mixture of 80% dichloromethane and 20% ethanol (w/w) (solid concentration: 8 w/w%). The obtained solution was subjected to spray drying with an atomizer under the following conditions, thereby obtaining an amorphous solid dispersion (specifically spray-dried powder).
- Flow rate (mL/min): 8
- Atomizing air (MPa): 0.15
- Inlet temperature (°C): 75
- Drying Air (m3/min): 0.45
- Overnight vacuum drying at 50°C

The following enteric polymers were used.
HPMC AS-LF (hypromellose acetate succinate: Shin-Etsu Chemical Co., Ltd., AQOAT) (dissolved at a pH of 5.5 or higher)
HPMC AS-MF (hypromellose acetate succinate: Shin-Etsu Chemical Co., Ltd., AQOAT) (dissolved at a pH of 6.0 or higher)
HPMC AS-HF (hypromellose acetate succinate: Shin-Etsu Chemical Co., Ltd., AQOAT) (dissolved at a pH of 6.8 or higher)
TC-5R (hypromellose: Shin-Etsu Chemical Co., Ltd.) (viscosity of the 2% aqueous solution at 20°C (Japanese Pharmacopoeia): about 6 mPa·s)
HP-50 (hypromellose phthalate: Shin-Etsu Chemical Co., Ltd.) (dissolved at a pH of 5.0 or higher; viscosity of the 10% solution in a methanol-dichloromethane mixture (1:1) at 20°C (Japanese Pharmacopoeia): about 55 mPa·s)
HP-55 (hypromellose phthalate: Shin-Etsu Chemical Co., Ltd.) (dissolved at a pH of 5.5 or higher; viscosity of the 10% solution in a methanol-dichloromethane mixture (1:1) at 20°C (Japanese Pharmacopoeia): about 40 mPa·s)
K-25 (polyvinylpyrrolidone: BASF Kollidon 25)

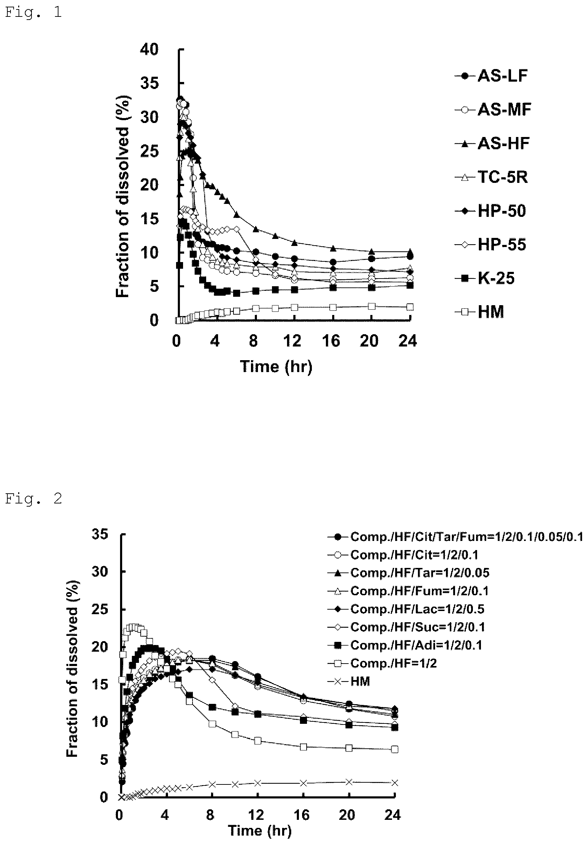

The release rate of compound (I) from each of the obtained amorphous solid dispersions was evaluated by measuring the dissolution rate of compound (I) at one-hour intervals for 24 hours. The dissolution test was performed in accordance with the paddle method specified in the dissolution test section of the Japanese Pharmacopoeia, Seventeenth Edition. The test was performed by using 900 mL of the second fluid for dissolution test (pH: approximately 7, potassium dihydrogen phosphate and disodium hydrogen phosphate) described in the Japanese Pharmacopoeia as a test solution, at 37°C at a paddle rotation rate of 50 rpm. Sampling was performed over time, and compound (I) in the sampling solution was quantified with a UV detector (absorbance measurement wavelength: 323 nm and 380 nm). The first wavelength (323 nm) was set as the wavelength at which the absorbance of the main drug could be maximally detected, and the second wavelength (380 nm) was set as the wavelength at which the absorbance derived from the main drug was not detected. The dissolution rate was defined as the proportion (%) of the mass of dissolved compound (I) of the total amount (mass) of compound (I) contained in each amorphous solid dispersion taken as 100%. Compound (I) itself, which was not made into an amorphous solid dispersion (hammer-milled product; denoted as "HM"), was also examined for dissolution rate by performing the dissolution test. Fig. 1 shows the results.

The results indicate that the amorphous solid dispersion containing compound (I) prepared by using any of the enteric polymers exhibits excellent dissolution.

In the following study, HPMC AS-HF was used for the enteric polymer for preparing an amorphous solid dispersion, unless otherwise indicated.

### Study of Stability of Compound (I) in Amorphous Solid Dispersion

To examine the stability of compound (I) contained in an amorphous solid dispersion prepared by using HPMC AS-HF as an enteric polymer, the amount of a degradation product of compound (I) generated after storage was measured.

Specifically, after an amorphous solid dispersion was stored at 40°C for 4 weeks under closed conditions, the amount of generated compound (D) (8-(1-benzothiophen-4-yl)-8-aza-5-azoniaspiro[4.5]decane), which is one of the degradation products of compound (I) (brexpiprazole) was examined by HPLC.

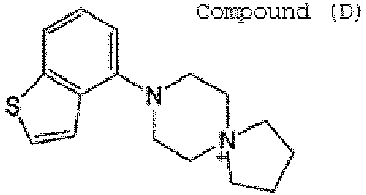

More specifically, 50 mg of an amorphous solid dispersion in terms of compound (I) was weighed, and a 50% MeCN/50% MeOH solution was added to completely dissolve the dispersion and to make the solution 100 mL. 10 mL of the solution was taken and made up to 25 mL by adding a 1% acetic acid solution. After being filtered through a 0.45-um filter, 50 µL of this solution was injected into HPLC. The measurement conditions for HPLC were as follows.
HPLC: LC-2010C (Shimadzu)
Measurement wavelength (nm): 274
Analysis column: Capcell Pak C18, MGII 3 pm, 4.6 mm I.D. × 150 mm (Shiseido)
Flow rate (mL/min): 1.7
Mobile phase: 0.01 mol/L sodium sulfate
solution/acetonitrile/methanol/acetic acid = 140/45/15/2
Injection amount (µL): 50
Measurement time (min): 30

The proportion of the area of the peak area of compound (D) in the obtained HPLC chromatogram was calculated based on the total area of the peak area of compound (I) and the peak area of the degradation products of compound (I) taken as 100%.

The results of the above study indicated that compound (D) formed in an amount of 0.14%.

Subsequently, various organic acids were further dissolved in the solution, and spray drying was performed under the same conditions as above to prepare amorphous solid dispersions. To examine the stability, the obtained amorphous solid dispersions were stored at 40°C for 4 weeks under closed conditions, and then the amount of generated compound (D) was measured by HPLC under the same conditions as above.

The solutions were prepared by dissolving 6 g of compound (I), 12 g of an enteric polymer (HPMC AS-HF), and 0.6 g, 0.3 g, or 0.06 g of various organic acids in 213.9 g or 210.5 g of a mixture of 80% dichloromethane and 20% ethanol (w/w). As for the dissolution order, an organic acid was completely dissolved in ethanol, then dichloromethane was added and mixed, and an enteric polymer (HPMC AS-HF) was further dissolved therein, followed by finally adding and dissolving compound (I).

However, in the preparation of a solution in which lactic acid was dissolved as an organic acid, the composition of the solution was changed: 20 g of compound (I), 40 g of an enteric polymer (HPMC AS-HF), 10 g of lactic acid (50% of compound (I)) or 0.2 g of lactic acid (1% of compound (I)) were dissolved in 430 g of a mixture of 80% dichloromethane and 20% ethanol (w/w) (the dissolving order was the same as above).

In the preparation of a solution in which citric acid, tartaric acid, and fumaric acid were dissolved as organic acids, the composition of the solution was changed: 16 g of compound (I), 32 g of an enteric polymer (HPMC AS-HF), and various organic acids (citric acid 1.6 g, tartaric acid 0.8 g, fumaric acid 1.6 g) were dissolved in 912 g of a mixture of 60% dichloromethane and 40% ethanol (w/w) (the dissolving order was the same as above). Additionally, in this case only, the spray-drying conditions were changed as given below.
· Flow rate (mL/min): 10
· Atomizing air (MPa): 0.15
· Inlet temperature (°C): 80
· Outlet temperature (°C): 49 to 50
· Drying air (m³/min): 0.40
· Overnight vacuum drying at 50°C

The following table shows the amount of compound (D) in the amorphous solid dispersions obtained by using various organic acids after storage at 40°C for 4 weeks under closed conditions. In the table, the amount of each organic acid (%) is a percentage of the mass of the organic acid contained in the individual solutions based on 100% of the mass of compound (I) contained in the solutions subjected to spray drying.

The lactic acid used as a starting material was in liquid form with a purity of 85 to 92%, containing about 10% of water as an impurity. The spray drying treatment eliminates water, and the remaining lactic acid content after preparation is about 8.8 g to 10 g. The same is true in the following study.

**Table 1**

| Organic Acid | Amount (%) | Compound (D) (%) |
|---|---|---|
| None | 0 | 0.14 |
| Citric Acid | 10 | 0.08 |
| Tartaric Acid | 1 | 0.09 |
| Tartaric Acid | 10 | 0.07 |
| Succinic Acid | 10 | 0.1 |
| Adipic Acid | 5 | 0.1 |
| Fumaric Acid | 10 | 0.08 |
| Lactic Acid | 1 | 0.06 |
| Lactic Acid | 50 | 0.03 |
| Citric Acid + Tartaric Acid + Fumaric Acid | 10+5+10 | 0.03 |

The amorphous solid dispersions obtained by using various organic acids were found to exhibit a decrease in degradation of compound (I), which is caused by long-term storage. Additionally, all of the amorphous solid dispersions obtained in this study stably remained amorphous after long-term storage. In other words, the over-time stability of compound (I) contained in each of the amorphous solid dispersions obtained by using various organic acids was found to have improved. This stability was considered satisfactory in light of the revised version (*Iyakushinpatsu* No. 0624001, June 24, 2003) of the guidelines for impurities in drug products among drugs containing new active ingredients (*Yakushin* No. 539, June 23, 1997, issued by the Director of the Pharmaceutical Affairs Bureau), which were established based on the ICH standards.

The release rate of compound (I) from each of the amorphous solid dispersions obtained by using various organic acids was evaluated by measuring the dissolution rate of compound (I) at one-hour intervals for 24 hours. The dissolution test was performed in the same manner as above. Fig. 2 shows the results. In Fig. 2, "Comp." denotes compound (I). "HF" also denotes HPMC AS-HF. "Cit," "Tar," "Fum," "Lac," "Suc," and "Adi" respectively denote citric acid, tartaric acid, fumaric acid, lactic acid, succinic acid, and adipic acid. "HM" denotes compound (I) itself (hammer-milled product) that was not formed into an amorphous solid dispersion.

As can be seen from the results, the amorphous solid dispersions obtained by using various organic acids exhibited an improved dissolution rate compared with the amorphous solid dispersion obtained without using an organic acid (Comp./HF). In particular, the dissolution rate was notably improved when lactic acid or an organic acid (citric acid, tartaric acid, and fumaric acid) was combined.

### Analysis of Amorphous Solid Dispersion by X-ray Diffractometry (XRD)

Solution (i) obtained by dissolving 12 g of compound (I) in 162 g of a mixture of 70% dichloromethane and 30% ethanol (w/w) or solution (ii) obtained by dissolving 12 g of compound (I) and 6 g of lactic acid in 162 g of a mixture of 70% dichloromethane and 30% ethanol (w/w) was subjected to spray drying in the same manner as above, thereby obtaining amorphous solid dispersion (i) or amorphous solid dispersion (ii).

Amorphous solid dispersions (i) and (ii) were analyzed by X-ray diffractometry immediately after preparation and after storage at 40°C under closed conditions for 3 days since preparation.

The conditions for X-ray diffractometry were as follows.
Model: X'Pert Pro MPD (Spectris)
Voltage (kV): 45
Current (mA): 40
Configuration: Spinner reflect-trans
Scan axis: 2 theta
Start angle: 3.000
End angle: 40.000
Step size: 0.0167113
Time per step: 3.175
Scan speed (°/s): 0.668451
Wobble axis: omega
Number of steps: 5
Step size: 1.000

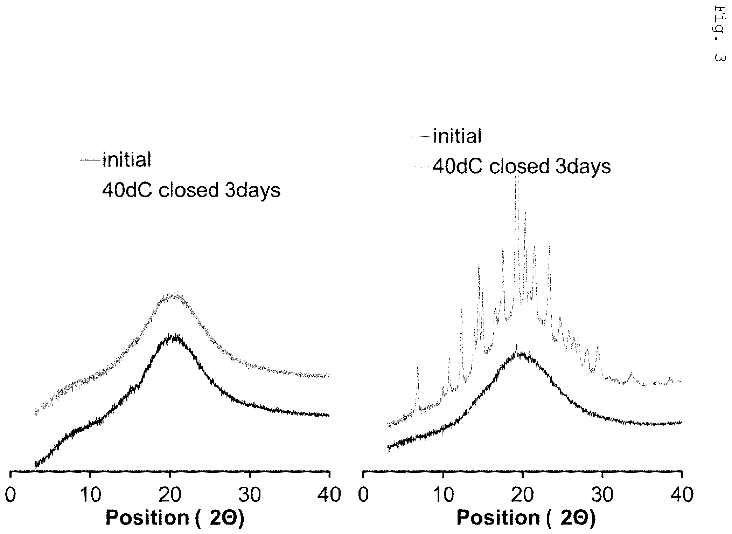

Fig. 3 shows the analysis results. Fig. 3 shows the results of amorphous solid dispersion (i) on the right and the results of amorphous solid dispersion (ii) on the left. Amorphous solid dispersion (i) showed many peaks after storage at 40°C for 3 days, suggesting that some of the amorphous form transitioned to crystals. On the other hand, amorphous solid dispersion (ii) did not show changes in pattern even after storage at 40°C for 3 days, suggesting that amorphous solid dispersion (ii) stably remained amorphous.

### Thermal Analysis of Amorphous Solid Dispersion (TGA and DSC)

Amorphous solid dispersion (ii) was subjected to thermogravimetric measurement (TGA) and differential scanning calorimetry (DSC).

The following instruments were used for measurements. The amount of the sample (amorphous solid dispersion (ii)) was about 5 mg in both measurements.
TGA: TGA Q5000 (TA Instruments)
DSC: DSC Q2000 (TA Instruments)

In TGA analysis, the temperature was increased to 250°C at 10°C/min. In DSC analysis, the temperature was first increased to 100°C at 10°C/min to volatilize the dichloromethane and ethanol thought to remain in trace amounts and then decreased to 25°C at 10°C/min, followed by increasing it again to 150°C at 10°C/min.

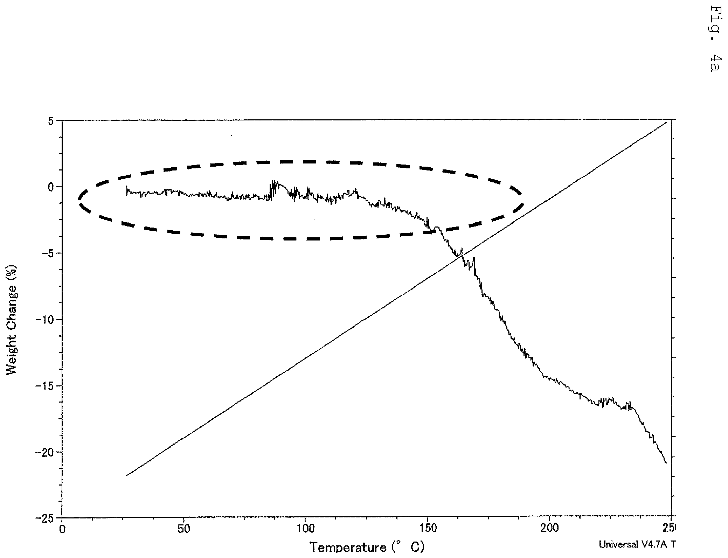

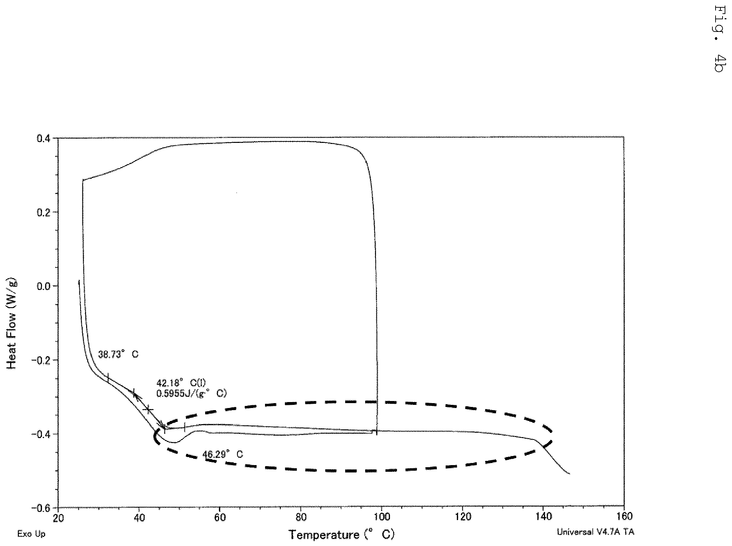

Fig. 4a (TGA analysis) and Fig. 4b (DSC analysis) show the results. In Fig. 4a, no peaks indicating heat generation were measured (see the area circled with a dotted line). In Fig. 4b, no peaks indicating weight change were also measured (see the area circled with a dotted line). These also suggest that amorphous solid dispersion (ii) did not transition to crystals (remained amorphous), thus demonstrating that amorphous solid dispersion (ii) was stable.

### NMR Measurement of Amorphous Solid Dispersion

Amorphous solid dispersion (ii) was analyzed by NMR. NMR analysis was also performed in the same manner for compound (I) itself, which was not formed into an amorphous solid dispersion, HPMC AS-HF, and lactic acid. Further, NMR analysis was also performed in the same manner for an amorphous solid dispersion prepared from a solution of HPMC AS-HF and lactic acid ("amorphous solid dispersion (iii)"). The solution of HPMC AS-HF and lactic acid was a solution of 40 g of HPMC AS-HF and 10 g of lactic acid in 430 g of a mixture of 70% methylene chloride and 30% ethanol (w/w).

The NMR measurement conditions are shown below.
Measuring instrument: ECA-500 (JEOL RESONANCE, Inc.)
Observed nucleus: ¹H
Sample amount: about 5 mg
Solvent: DMSO-d₆
Reference value for chemical shift: 2.49 ppm (DMSO)
Cumulated number: 8

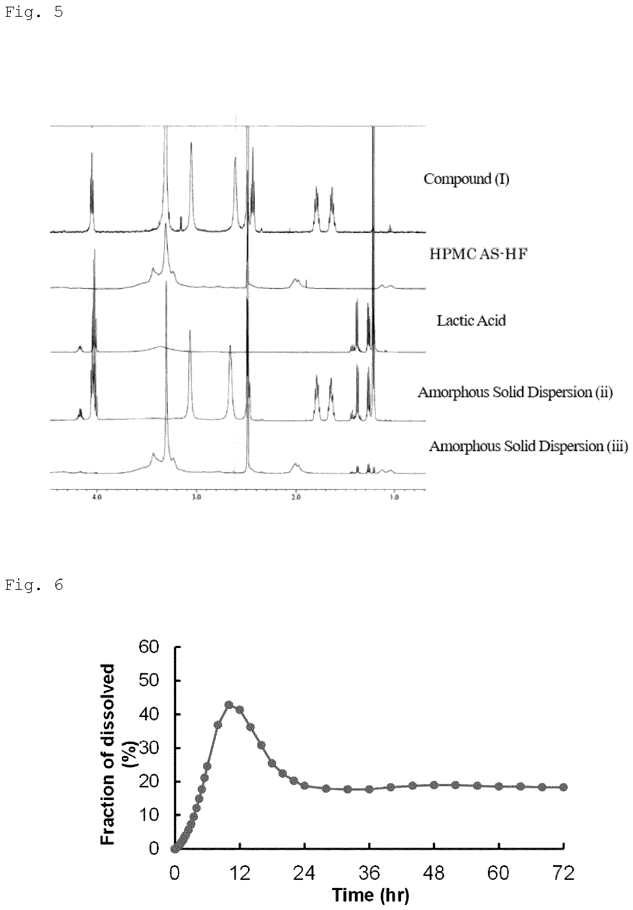

Fig. 5 shows the results. In Fig. 5, peaks are misaligned between compound (I) and amorphous solid dispersion (ii) (see the dotted line in the figure). This indicates that compound (I) and lactic acid in amorphous solid dispersion (ii) molecularly interacted. The amorphous form in amorphous solid dispersion (ii) was then found to be formed of compound (I) and lactic acid.

### Preparation and Examination of Tablets

A sustained-release hydrogel formulation containing amorphous solid dispersion (ii) (hydrogel matrix tablet) was produced according to a known commonly used production process. Specifically, hydrogel matrix tablets (uncoated tablets) were prepared by mixing and tableting ingredients so that 41.4 mg of amorphous solid dispersion (ii) (containing 12 mg of compound (I)), 80 mg of hypromellose, 60 mg of sodium starch glycolate, and 0.6 mg of magnesium stearate were contained per tablet. Hydrogel matrix tablets are a known technique for controlling drug release with a hydrogel formed from water absorbed from the alimentary tract (after a coating film is dissolved due to a rise in pH after gastric excretion, if the tablets are coated).

Additionally, enteric coating was applied to the hydrogel matrix tablets (uncoated tablets) to prepare an enteric-coated drug. Specifically, tablets containing 9.1 mg of a methacrylate copolymer LD (Eudragit L 30 D-55, Evonik), 4.55 mg of talc, and 0.91 mg of triethyl citrate, per tablet, were subjected to enteric coating in accordance with an ordinary method by using a general-purpose coater. Thereafter, color coating was applied by using OPADRY 03A420002 (5.4 mg), thereby obtaining enteric-coated tablets.

The release rate of compound (I) from the obtained enteric-coated tablets was evaluated by measuring the dissolution rate of compound (I) at one-hour intervals for 24 hours. The dissolution test was performed in the same manner as above. Fig. 6 shows the results. The enteric-coated drug exhibited an excellent supersaturated dissolution profile.

### Evaluation of Blood Concentration of Compound (I) in Peroral Administration to Human

The blood concentration of compound (I) in peroral administration of the oral pharmaceutical composition of the present disclosure to humans was evaluated based on the following single-dose protocol. The formulation used in this evaluation (test formulation) was a hydrogel matrix tablet prepared in accordance with the present disclosure, containing an amorphous solid dispersion of 12 mg of compound (I) (active ingredient). In the single-dose protocol, a single dose of a conventional tablet of compound (I) (a tablet containing compound (I) not formed into an amorphous solid dispersion) was administered. After cessation, a single dose of 12 mg of a test formulation (a single 12-mg tablet) was administered. The PK parameters of compound (I) were analyzed. The median Tmax (the time it takes for a drug to reach the maximum plasma concentration) was prolonged compared with the conventional tablet. These results appear to indicate that the oral pharmaceutical composition of the present disclosure is able to maintain the blood concentration of compound (I) desirable for a once-weekly formulation (e.g., 15 ng/mL to 400 ng/mL at steady state).

## Claims

1. An amorphous form comprising 7-[4-(4-benzo[b]thiophen-4-yl-piperazin-1-yl)butoxyJ-1H-quinolin-2-one and at least one organic acid.

2. The amorphous form according to claim 1, wherein the organic acid is a carboxylic acid.

3. The amorphous form according to claim 1 or 2, wherein the organic acid is lactic acid.

4. An amorphous solid dispersion comprising the amorphous form of any one of claims 1 to 3 and at least one enteric polymer.

5. An amorphous solid dispersion comprising 7-[4-(4-benzo[b]thiophen-4-yl-piperazin-1-yl)butoxy]-1H-quinolin-2-one and at least one enteric polymer.

6. The amorphous solid dispersion according to claim 4 or 5, wherein the enteric polymer is a nonionic water-soluble polymer.

7. The amorphous solid dispersion according to any one of claims 4 to 6, wherein the enteric polymer is at least one selected from the group consisting of hydroxypropyl methylcellulose, derivatives thereof, and polyvinylpyrrolidone.

8. The amorphous form according to any one of claims 1 to 3, or the amorphous solid dispersion according to any one of claims 4 to 7, which is a spray-dried powder.

9. A method for producing the amorphous form of any one of claims 1 to 3, or the amorphous solid dispersion of any one of claims 4 to 8, the method comprising
subjecting to spray drying a mixture containing
7-[4-(4-benzo[b]thiophen-4-yl-piperazin-1-yl)butoxyJ-1H-quinolin-2-one, and
at least one selected from the group consisting of an enteric polymer and an organic acid.

10. A pharmaceutical composition comprising
at least one selected from the group consisting of the amorphous form of any one of claims 1 to 3 and the amorphous solid dispersion of any one of claims 4 to 8, and
a hydrophilic polymer.

11. The pharmaceutical composition according to claim 10, wherein the hydrophilic polymer is at least one selected from the group consisting of cellulose-based water-soluble polymers, polyalkylene oxides, polyalkylene glycols, and polyvinyl alcohols.

12. The pharmaceutical composition according to claim 10, or 11, which is an oral solid pharmaceutical composition.

13. The pharmaceutical composition according to claim 12, wherein after oral administration of the pharmaceutical composition to a human, the steady-state blood concentration of the 7-[4-(4-benzo[b]thiophen-4-yl-piperazin-1-yl)butoxy]-1H-quinolin-2-one is maintained within the range of 15 ng/mL to 400 ng/mL for 1 week.

14. The pharmaceutical composition according to any one of claims 9 to 13, for use in the administration of the 7-[4-(4-benzo[b]thiophen-4-yl-piperazin-1-yl)butoxy]-1H-quinolin-2-one at a dose of 5 mg to 60 mg once a week.

15. The pharmaceutical composition according to any one of claims 9 to 14, for use in the prevention or treatment of a central neurological disease.
